Europäisches Patentamt

European Patent Office

Office européen des brevets

(11) Veröffentlichungsnummer: **0 196 731**

**A1**

(12) ## EUROPÄISCHE PATENTANMELDUNG

(21) Anmeldenummer: 86200566.7

(22) Anmeldetag: 19.03.86

(51) Int. Cl.⁴: **G 01 N 33/549**
**G 01 N 33/52**

(30) Priorität: 27.03.85 DE 3511012

(43) Veröffentlichungstag der Anmeldung:
08.10.86 Patentblatt 86/41

(84) Benannte Vertragsstaaten:
AT BE CH DE FR GB IT LI LU NL SE

(71) Anmelder: BEHRINGWERKE Aktiengesellschaft
Postfach 1140
D-3550 Marburg 1(DE)

(72) Erfinder: Pauly, Hans Erwin, Dr.
Finkenstrasse 1
D-3563 Dautphetal 2(DE)

(74) Vertreter: Meyer-Dulheuer, Karl-Hermann, Dr. et al,
HOECHST Aktiengesellschaft Zentrale Patentabteilung
Postfach 80 03 20
D-6230 Frankfurt/Main 80(DE)

(54) **Verfahren und Testvorrichtung zur Bestimmung von Analyten.**

(57) Die Erfindung betrifft ein Verfahren zum Nachweis eines Analyten mit bioaffinen Eigenschaften, der in Gegenwart von partikulären Reaktionspartnern Aggregate oder Agglutinate verursacht oder verhindert. Nach Abtrennung der Agglutinate werden die nicht aggregierten Partikel mit Hilfe eines signalproduzierenden Systems (SPS) nachgewiesen.

Die Erfindung betrifft weiterhin ein diagnostisches Mittel oder eine diagnostische Testvorrichtung bestehend aus flächenförmigen Elementen, die über ihre Kanten nebeneinander oder über ihre Flächen übereinander angeordnet sind und miteinander in Kontakt stehen. Die Elemente bestehen aus Materialien mit den Eigenschaften, wässrige Flüssigkeiten und darin dispergierte Partikel aufzunehmen und zu transportieren und für agglutinierte Partikel in der Flüssigkeit undurchlässig zu sein.

FIG.1

Verfahren und Testvorrichtung zur Bestimmung von
Analyten

---

Die Erfindung betrifft ein Verfahren zur Auswertung von
Agglutinationen verursacht durch Komplexierung bioaffiner
Bindungspartner unter Zuhilfenahme eines signalproduzierenden Systems. Diese Erfindung betrifft weiterhin eine
Testvorrichtung, mit deren Hilfe das Verfahren durchgeführt wird.

Die überwiegende Zahl von Bindungsreaktionen zwischen
bioaffinen Bindungspartnern führt zu einem Komplex, der
eine größere räumliche Ausdehnung als jeder der einzelnen Bindungspartner besitzt. Besonders ausgeprägt ist
diese Größenänderung, wenn ein Bindungspartner des bioaffinen Bindungspaares von Natur aus partikulär ist oder
an ein dispergierbares Partikel oder an ein großes, lösliches Polymermolekül geknüpft wird. Die vor der Bindungsreaktion als Monomere vorliegenden Partikel bilden
durch die Bindungsreaktion dimere oder oligomere Aggregate. Beispiele hierfür sind die Bakterienagglutinationen,
die Bentonit-Agglutination, Haemagglutination und die
Latex-Agglutination.

In analoger Weise wird in einem in der PCT WO 83/04314
beschriebenen Latex-Agglutinations-Inhibitionstest zur
Bestimmung von Haptenen, z.B. Penicillin, eine Trennung
zwischen immunchemisch aggregierten und nicht-aggregierten Latex-Partikeln erzielt. Dabei wird sensibilisierter
Latex in einer Spritze mit einer Penicillin-haltigen
Probe gemischt, inkubiert und unter Druck durch eine
vorbefeuchtete Filtrationsmembran gepreßt und der nicht aggregierte Anteil in einem Spektrophotometer anhand der Lichtstreueigenschaft bestimmt.

Nachteilig ist bei diesem Verfahren die Vielzahl an Operationen, die vom Anwender verlangt werden, wie z.B. Dosieren und Mischen von Reagenzien und Proben, exakte Einhaltung von Inkubationszeiten, Druckfiltration, Auffangen des Filtrats, Bestimmung des Anteils an aggregierten oder nicht-aggregierten Partikeln. Neben dem entsprechenden manuellen Aufwand ist zusätzlich die Möglichkeit von Bedienungsfehlern zu sehen, die das Ergebnis des Bestimmungsverfahrens verfälschen. Insbesondere die Trennung der aggregierten und nicht-aggregierten Partikel an einer Filtermembran in reproduzierbarer Weise ist außerordentlich schwierig, da es in Abhängigkeit vom angewandten Druck zu einer Anreicherung von Partikeln in Membrannähe kommt, die das Filtrationsverhalten entscheidend beeinflußt. Entsprechend passieren zu unterschiedlichen Zeiten des Filtrationsvorganges unterschiedliche Mengen an nicht-aggregierten Partikeln die Membran, so daß die Entnahme einer repräsentativen Menge an Filtrat für das anschließende Bestimmungsverfahren schwierig wird.

Es wurde ein Verfahren gefunden, mit dem die durch bioaffine Wechselwirkung erzeugten Agglutinate von Partikeln durch selbsttätige Filtration nach Art einer Chromatographie von den dispergiert gebliebenen Partikeln getrennt werden und der Anteil der dispergierten Partikel mit Hilfe eines signalproduzierenden Systems (SPS) nachgewiesen wird. Das Verfahren wird durchgeführt unter Benutzung einer Vorrichtung, die eine selbsttätige Filtration nach Art einer Chromatographie ermöglicht.

Gegenstand der Erfindung ist ein Verfahren zum Nachweis und zur Bestimmung der Konzentration eines in wässriger Lösung vorliegenden Analyten mit bioaffinen Bindungseigenschaften durch Agglutination oder durch Agglutinationshemmung von Partikeln, welche mit bioaffinen Bindungseigenschaften und zugleich mit einem SPS oder mit
Teilen eines SPS ausgestattet sind, dadurch gekennzeichnet, daß

a) eine Trennung von agglutinierten und nicht agglutinierten Partikeln vorgenommen wird mit Hilfe einer
Vorrichtung bestehend aus Materialien, die wässrige
Lösungen und darin dispergierte Partikel aufsaugen
und transportieren und

b) der abgetrennte Anteil von nicht agglutinierten Partikeln über sein SPS bestimmt wird.

Die bei dem Verfahren verwendeten Partikel enthalten
neben den Komponenten, die ihnen ihre bioaffinen Bindungseigenschaften verleihen, zusätzlich mindestens
einen Bestandteil des SPS.

Das Verfahren wird durchgeführt mit Hilfe einer Vorrichtung, die nach Art einer Chromatographie die selbsttätige Abtrennung von agglutinierten und dispergierten Partikeln ermöglicht unter Verwendung von Materialien,
welche wässrige Dispersionen aufsaugen und transportieren, Agglutinate jedoch zurückhalten. Dabei wird die den
Analyten enthaltende Lösung auf einen der Bereiche des
Mittels, die als Auftrags- oder Reaktionszonen ausgebildet sind, aufgetragen. Der Analyt reagiert entweder mit
den in der Reaktionszone in fester Form enthaltenen oder
auf die Trennzone als Dispersion aufgebrachten Partikeln
und gegebenenfalls mit den übrigen, zuvor beschriebenen
Komponenten des bioaffinen Bindungssystems. Die sich
bildenden Agglutinate wurden an oder in einem Bereich
des Mittels, der als Trennzone ausgebildet ist, festgehalten. Die als Dispersion verbliebenen Partikel wandern mit der Flüssigkeit durch die Trennzone und gelangen in einen als Nachweiszone ausgebildeten Bereich, wo sie unter Mitwirkung aller Komponenten des SPS ein auswertbares Signal erzeugen.

Gegenstand der Erfindung ist weiterhin eine Vorrichtung zum Nachweis und zur Bestimmung der Konzentration eines in wässriger Lösung vorliegenden Analyten mit bioaffinen Bindungseigenschaften mittels Partikeln in einer Agglutinationsreaktion, wobei agglutinierte und nicht agglutinierte Anteile voneinander getrennt werden, dadurch gekennzeichnet, daß

a) die Vorrichtung eine Auftrags-, eine Reaktions-, eine Trenn- und eine Nachweiszone enthält,

b) alle Zonen aus Materialien bestehen, die wässrige Flüssigkeiten aufnehmen und transportieren,

c) alle genannten Zonen für die nicht agglutinierten Partikel durchlässig sind,

d) zumindest die Trennzone für agglutinierte Partikel undurchlässig ist,

e) die Vorrichtung ein signalproduzierendes System enthält, welches in der Nachweiszone ein auswertbares Signal erzeugt.

Die Vorrichtung kann aus mehreren flächenförmigen Gebilden oder Plättchen bestehen, die miteinander in Kontakt stehen oder in Kontakt gebracht werden können, sich mit ihren Flächen kleinster Ausdehnung berührend, nebeneinander, also flächenförmig, oder sich mit ihren Flächen größter Ausdehnung berührend, also schichtförmig, angeordnet sind. Eine Kombination von flächen- und schichtförmiger Anordnung ist ebenfalls möglich. Die Plättchen
können durch Halterungen oder durch Träger in den beschriebenen Anordnungen befestigt sein. Die Plättchen
bestehen aus Materialien, die wässrige Lösungen und darin dispergierte Partikel aufnehmen und transportieren.

Wenn die Vorrichtung beispielsweise aus drei Plättchen
besteht, sind diese als Reaktions-, Trenn- und Nachweiszone ausgebildet, besteht sie aus vier Plättchen, so ist
das zusätzliche Plättchen als Auftragszone ausgebildet.

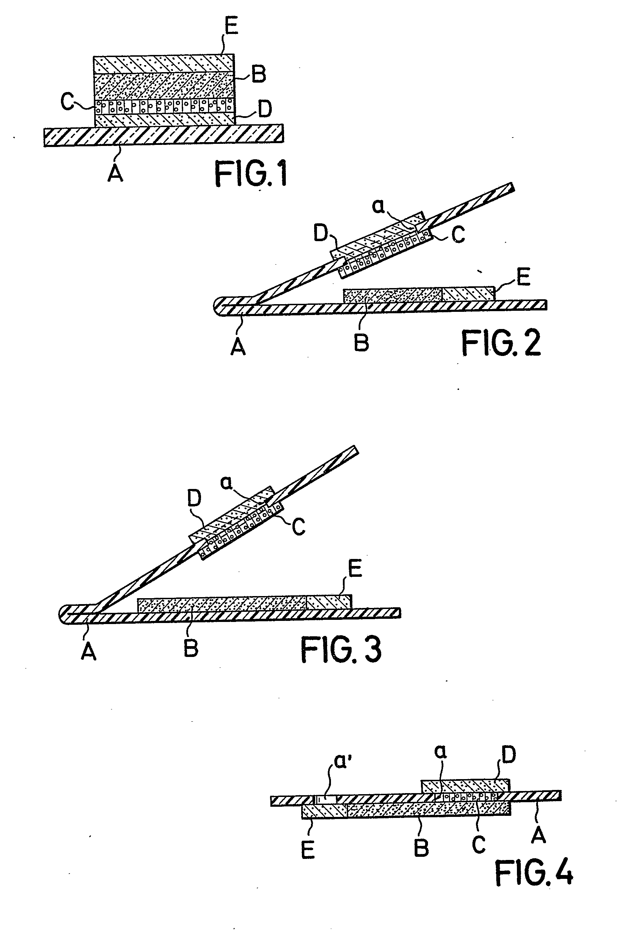

Beispielhafte Ausführungsformen der Vorrichtung sind in
Längsschnitten in Figuren 1, 2, 3 und 4 aufgezeigt. Die
Halterungen oder die Träger sind mit A gekennzeichnet.
Sie bestehen aus flächenförmigen Gebilden, aus Platten
oder Streifen, gefertigt aus Materialien, die keine
wässrigen Lösungen aufnehmen. Bei der in Fig. 1 dargestellten Vorrichtung ist der Träger eine transparente
Platte. Bei den in den Fig. 2, 3 und 4 dargestellten
Vorrichtungen sind die Träger nicht transparent, jedoch
mit Aussparungen oder Schlitzen a versehen. E kennzeichnet die Plättchen, ausgestattet als Auftrags-, B diejenigen, ausgestattet als Reaktions-, C solche, ausgestattet als Trenn- und D solche, ausgestattet als Nachweiszonen. Bei den Vorrichtungen gemäß Fig. 2 oder 3 besteht
die Halterung aus zwei flügelförmigen Streifen oder Platten, die an jeweils einer Kante miteinander verbunden
sind und die bei einer Verwendung aufeinander geklappt
werden. Der eine Flügel enthält auf seiner Oberseite in
flächenförmiger Anordnung, d.h. nebeneinander liegend,
Auftragszone E und Reaktionszone B, der andere Flügel in
schichtförmiger Anordnung auf seiner Unterseite und in
die Aussparung nach oben hineinreichend die Trennzone C,
auf seiner Oberseite und in die Aussparung nach unten
hineinreichend die Nachweiszone D, die Trennzone C berührend. Fig. 2 und Fig. 3 unterscheiden sich durch unterschiedlich große Auftrags- bzw. Reaktionszonen E bzw. B.

Fig. 4 zeigt eine Vorrichtung, bei der auf der Unterseite des Trägers A die Auftrags- und die Reaktionszone E und B flächenförmig nebeneinander angeordnet sind. Die Auftragszone E ist durch die Aussparung a' für den Probenauftrag und gegebenenfalls für den Auftrag von weiteren Lösungen zugänglich. In die Aussparung a ist die Trennzone C eingearbeitet und darüber die Nachweiszone D auf der Oberseite des Trägers A, die Trennzone C mit der Unterseite berührend, angebracht.

Im Falle des Vorhandenseins einer Auftragszone E dient sie zur Aufnahme der Lösung des Analyten. Sie kann zugleich zur Aufnahme einer Lösung mit Bestandteilen des SPS und/oder einer Dispersion von Partikeln für die Agglutinationsreaktion sowie eines zusätzlichen Elutionsmittels dienen.

Die Reaktionszone B dient zur Bildung oder zur Inhibierung der Agglutination der Partikel unter Beteiligung aller Komponenten des bioaffinen Bindungssystems als auch gegebenenfalls zur Aufnahme einer Dispersion von Partikeln und der Lösung des Analyten, wenn keine Auftragszone (E) vorhanden ist. Sie kann auch die Partikel sowie lösliche Komponenten des bioaffinen Bindungssystems und des SPS in trockener Form enthalten.

Die Trennzone C dient zur Trennung der agglutinierten Partikel, die in der Reaktionszone entstehen. Die Agglutinate wurden entweder an der Grenzfläche von Reaktions- und Trennzone zurückgehalten oder von ihr aufgenommen und festgehalten. Sie ist durchlässig für die nicht agglutinierten Partikel und für alle übrigen löslichen Komponenten, die für die Bestimmung des Analyten erforderlich sind. Die Trennzone kann aus Materialien mit fasriger als auch mit kugeliger Struktur hergestellt sein, also aus Materialien mit anisotrop als auch mit isotrop porösen Strukturen.

Die Nachweiszone D dient zur Aufnahme der nach erfolgter bioaffiner Reaktion dorthin gewanderten, dispergiert gebliebenen Partikel und deren Nachweis mit Hilfe des SPS. Sie ist vorzugsweise aus Materialien gefertigt, die einen hohen Remissionsgrad für eingestrahltes Licht besitzen. Ist beispielsweise die Signalgebereinheit ein Chromophor oder Fluorophor, so werden keine weiteren Komponenten für das SPS benötigt, der Nachweis erfolgt durch visuelle Bewertung, eventuell anhand einer Farbvergleichsskala oder durch Reflektionsphotometrie oder -fluorometrie. In der aufgrund der erreichbaren Sensitivität bevorzugten Ausführungsform des Bestimmungsverfahrens, in der die Signalgebereinheit ein Enzym ist, wird in der Detektionszone die Enzymaktivität des die Trennzone passierenden Anteils des SPS bestimmt. Dies kann durch Hinzufügen einer Enzym-Substrat-Lösung auf die Detektionszone erfolgen, bevorzugt enthält jedoch die Detektionszone alle Komponenten der Enzym-Substrat-Reaktion. Bei bestimmten Enzymen, z.B. Peroxidase, kann es aus Gründen der Reagenzstabilität erforderlich sein, die Detektionszone aus zwei Zonen herzustellen, die miteinander in saugfähiger Verbindung stehen, und in denen jeweils eine oder mehrere Komponenten der Substratreaktion getrennt von den anderen inkorporiert sind.

Als Partikel werden Latices mit hydrophiler Oberfläche bevorzugt, insbesondere solche mit Kern-Schale-Aufbau, wie in DOS 31 45 082 beschrieben. Andere in wässrigen Lösungen dispergierbare Partikel sind ebenfalls geeignet. An die Partikel sind mindestens eine Komponente des für den Analyten spezifischen bioaffinen Bindungssystems und mindestens eine Komponente des SPS gebunden. Bevorzugt werden kovalente Bindungen für die bioaffinen Bindungspartner und für die Komponenten des SPS, aber auch das Avidin/Streptavidin-Biotin-System ist für die Bindung von Komponenten des SPS an die Partikel geeignet. Gebundene Komponenten des SPS können Enzyme wie Glucoseoxidase, alkalische Phosphatase, Peroxidase oder Beta-Galactosidase sowie Chromophore oder Fluorophore.

Die Partner oder Komponenten des bioaffinen Systems, die bei Anwesenheit des Analyten an der Agglutination von Partikeln oder an deren Inhibierung mitwirken, sind sofern es sich nicht um die an die Partikel gebundenen Bindungspartner handelt, lösliche Komponenten, die in Vorrichtung in fester Form untergebracht sind oder in einer Lösung auf die zuvor beschriebene Auftrags- oder Reaktionszone aufgebracht werden können.

Eine Agglutination findet statt, wenn beispielsweise

a) die dispergierten Partikel einen mindestens bifunktionellen bioaffinen Bindungspartner des Analyten enthalten und

b) die dispergierten Partikel den Analyten oder ein bindungsfähiges Derivat des Analyten enthalten und ein mindestens bifunktioneller Bindungspartner des Analyten vorhanden ist.

Eine Hemmung der Agglutination durch den Analyten erfolgt, wenn die dispergierten Partikel einen mindestens bifunktionellen Bindungspartner des Analyten enthalten und ein mindestens bifunktionelles Derivat des Analyten vorhanden ist.

Die Erfindung wird durch die folgenden Beispiele näher erläutert ohne den Erfindungsgegenstand auf diese zu beschränken.

## Beispiel 1

Herstellung einer Testvorrichtung zum Nachweis von Antikörpern gegen Streptolysin-O (SL-O)

### 1.1. Latex-Partikel mit Kern-Schale-Aufbau Die Herstellung von zur kovalenten Bindung von Proteinen geeigneten Latex-Partikeln erfolgte, wie im Beispiel 2b der DE 31 45 082 beschrieben, unter Verwendung von Polystyrol-Latex-Kernen. Die nach einer Pfropfpolymerisation mit Styrol, Methacrylsäure und Methacrylamido-Acedaldehyd di-n-pentylacetal erhaltenen Latices mit Kern-Schale-Aufbau besaßen einen Partikeldurchmesser von 160 nm, der Gehalt an freisetzbaren Aldehydgruppen betrug 0,05 mval/g Latex.

### 1.2 1-"Peroxidasyl"-1,2,9,10-tetraza-3,8-dioxodecan 5 mg lyophilisierte Meerrettich Peroxidase (POD), 250 U/mg bei 25° C (Boehringer Mannheim, Reinheitsgrad I, Best.-Nr. 121606) wurden in 2 ml eines Puffers von 0,1 mol/l Natriumhydrogenphosphat, pH 6,0 gelöst, 0,5 ml einer frisch hergestellten Lösung von 50 mmol/l Natriummetaperjodat in Wasser zugesetzt und unter Rühren 10 min bei Raumtemperatur unter Lichtschutz inkubiert. Danach wurde der Reaktionsansatz von Perjodat befreit durch Gelfiltration an Sephadex G-25, äquilibriert mit obengenanntem Phosphatpuffer. Die Peroxidase-haltige Fraktion von 3 ml wurde mit einer Lösung von Adipinsäuredihydrazid in einer Endkonzentration von 0,1 mol/l 2 h bei Raumtemperatur in Gegenwart von 1 g/l Natriumcyanoborhydrid inkubiert. Anschließend wurde der Ansatz bei +4° C 2 Tage bei mehrfachem Pufferwechsel gegen obengenannten Phosphatpuffer dialysiert.

1.3   1-"Phosphatasyl"-1,2,9,10-tetraaza-3,8-dioxodecan 26 mg eines Lyophilisats von alkalischer Phosphatase aus Kälberdarm (100 U/mg Lyophilisat entsprechen ca. 1300 U/mg Protein bei 37° C - Boehringer Mannheim, Best.-Nr. 405639, Reinheitsgrad I) wurden analog zu Beispiel 1.2 mit Natriummetaperjodat und Adipinsäuredihydrazid umgesetzt mit der Veränderung, daß der genannte Phosphatpuffer auf pH 7,0 eingestellt worden war und die Dialyse vorgenommen wurde mit einem Puffer von 50 mmol/l Natriumborat, 10 mmol/l Magnesiumchlorid, 0,1 mol/l Zinkchlorid, eingestellt mit Natronlauge auf pH 8,0.

1.4   Bindung von 1-"Peroxidasyl"-1,2,9,10-tetraaza-3,8-dioxodecan und von Streptolysin-0 an Latex

10 ml einer 1%igen Suspension des nach Beispiel 1.1 hergestellten Latex wurden mit 0,5 ml 1 normaler HCl versetzt und 15 min bei Raumtemperatur inkubiert. Dann wurden 0,4 ml 1 normale NaOH, 1 ml einer gesättigten Natriumhydrogenphosphat-Lösung, pH 6,5, 8 ml einer 0,9%igen NaCl-Lösung, 200 µl der in Beispiel 1.2 erhaltenen Lösung von 1-"Peroxidasyl"-1,2,9,10-tetraaza-3,8-dioxodecan und 1 ml einer Lösung von 1 g/l Natriumcyanoborhydrid zugesetzt und 30 min bei Raumtemperatur inkubiert. Dazu wurden 0,4 ml einer wässrigen Lösung von 10 g/l Streptolysin-0 und 0,5 ml einer wässrigen Lösung

von 200 g/l Polyoxyethylensorbitanmonolaureat, bekannt unter dem Handelsnamen Tween® 20, zugesetzt und über Nacht bei Raumtemperatur inkubiert. Dann wurden 1 ml einer Lösung von 0,5 mol/l L-Asparaginsäure, die mit Natronlauge auf pH 6,5 eingestellt war, und 0,5 ml einer Lösung von 1 g/l Natriumcyanoborhydrid zugesetzt. Es wurde 1 h bei Raumtemperatur inkubiert und der Ansatz bei ca. 50.000 x g abzentrifugiert. Der Rückstand wurde in 20 ml 50 mmol/l Tris/HCl, pH 7,4 mit dem Zusatz von 1 g/l Humanserumalbumin resuspendiert. Nach wiederholter Zentrifugation wurde der Rückstand nochmals in 20 ml der Lösung von Tris/HCl, pH 7,4, mit dem Zusatz von Humanserumalbumin aufgenommen und mittels Ultraschall homogenisiert.

1.5 Herstellung einer Testvorrichtung Als inerter, wasserundurchlässiger Kunststoffträger wurde eine Polyesterfolie, Dicke 200 µm, Breite 8 cm, benutzt. Auf die Folie wurde im Abstand 2 cm von den beiden Seitenkanten jeweils ein doppelseitiges Klebeband (Fa. Beiersdorf, Hamburg) mit einer Breite von 0,8 cm angebracht. Diese Fläche der Folie wird damit zur Vorderseite erklärt. An einer Seitenkante wurde auch die Rückseite der Polyesterfolie in gleichem Abstand mit dem doppelseitigen Klebeband versehen. In diesen auf beiden Seiten von Klebeband bedeckten Streifen der Folie wurden sodann runde Löcher von 0,5 cm Durchmesser mit einem Abstand von Lochmitte zu Lochmitte von 0,8 cm gestanzt und auf die verbliebenen Klebeband-Teile ein 0,8 cm breiter Streifen eines Membranfilters, Typ RC-55, Porengröße 0,2 µm, der Fa. Sartorius, Hamburg, auf der Polyesterfolien-Vorderseite befestigt. Auf der zur Mitte gerichteten Seite des 2. Klebebandes auf dieser Vorderseite wurde ein 0,5 cm breiter Streifen von Chromatographie-Papier Nr. 1507 der Fa. Schleicher & Schuell, Dassel, fixiert, der die Reaktionszone darstellt und zuvor mit einer Lösung von 0,2 mg/ml Tetramethylbenzidin in Äthanol getränkt und vor dem Aufkleben unter Verwendung eines Ventilators getrocknet worden war. Das rückseitige Klebeband wurde ebenfalls mit einem 0,5 cm breiten Papierstreifen Nr. 1507 beklebt, der zuvor mit einer Lösung von 10 mmol/l eines äquimolaren Harnstoff.$H_2O_2$-Addukts in 50 mmol/l Citronensäure, eingestellt auf pH 5,0 mit einer gesättigten Lösung von $Na_2HPO_4$, getränkt und getrocknet worden war (Detektionszone). Mit einem Papierschneider wurde die Folienbahn quer zu den aufgeklebten Papierbahnen in Streifen von 8 mm Breite zerschnitten.

1.6  Bestimmung von Anti-Streptolysin-O (ASL-O) Das im Beispiel 1.5 beschriebene Testelement wurde zur ASL-O-Bestimmung eingesetzt. Dabei werden Antikörper gegen Streptolysin-O, einem Streptokokken-Antigen, detektiert. Dazu wurde ein Human-Immunglobulin-Präparat der Behringwerke, das den Handelsnamen Beriglobin® trägt, durch Verdünnen mit phosphatgepufferter physiologischer Kochsalzlösung auf einen Gehalt von 300 IU/ml, 150 IU/ml, 75 IU/ml usw. Anti-Streptolysin-O eingestellt. Davon wurden jeweils 50 µl auf einer schwarzen Glasplatte mit 50 µl der in 1.4. beschriebenen Dispersion von Latex an die Streptolysin-O und POD gebunden worden waren, gemischt. Dieser Ansatz wurde sofort aufgeteilt: 50 µl wurden auf die Reaktionszone des in 1.5. beschriebenen Testelementes gebracht und die restlichen 50 µl durch rotierendes Kippen der Glasplatte 1 min lang bewegt. Die Agglutinationsstärke des Latex auf der Glasplatte wurde visuell in üblicher Weise von 0 - +4 bewertet. Der auf die Testvorrichtung aufgetragene Anteil wurde 5 min in der Reaktionszone inkubiert, sodann wurde der Streifen umgeklappt und dadurch die Reaktions-, Trenn- und Detektionszone in saugfähige Verbindung gebracht. Die in der Detektionszone nach 5 min entstandene blaugrüne Färbung wurde durch Reflektionsphotometrie mit Hilfe eines Rapimat Reflektionsphotometers der Behringwerke AG Marburg, einem Teststreifenauswertegerät bestimmt.

Die erhaltenen Ergebnisse sind in Tabelle 1 gegenübergestellt.

Beispiel 2

Herstellung einer Latex-Partikel enthaltenden Testvorrichtung zur Bestimmung von Antikörpern gegen Streptolysin-O und Verwendung dieser Testvorrichtung zur Bestimmung der Antikörper

Mit der nach 1.4. hergestellten Dispersion von Latex, an die Peroxidase und Streptolysin-O gebunden waren, wurden auf einer Glasplatte ein Papiermaschinensieb mit einer Maschenzahl von 225/cm$^2$ (Fa. Kufferrath, Reutlingen) in einer Menge von 20 ml/cm$^2$ getränkt und anschließend lyophilisiert. Unter Verwendung eines doppelseitigen, 0,8 cm breiten Klebebandes (Fa. Beiersdorf, Hamburg) wurde bei der in 1.5. beschriebenen Testvorrichtung bündig zur Reaktionszone ein 0,8 cm breiter Streifen des lyophilisierten latexhaltigen Papiermaschinensiebs angebracht.

Auf diese Fläche wurden jeweils 50 µl der in 1.6. beschriebenen Anti-Streptolysin-O Verdünnungsreihe aufgetragen. Nachdem die Flüssigkeitsfront das Ende der
Reaktionszone erreicht hatte, wurde durch Umklappen die
Verbindung zur Detektionszone hergestellt. Die in der
Detektionszone nach 15 min entstandene Färbung wurde mit
dem schon in 1.6. genannten Reflektionsphotometer ausgewertet. Das Ergebnis ist in Tabelle 2 dargestellt.

0196731

Tabelle 1

| Anti-Streptolysin-O (IU/ml) | Agglutinations-stärke (visuelle Beurteilung) | Reflektro-metrie bei 660 nm (Bits) |
|---|---|---|
| 300 | +++ | 142 |
| 150 | ++++ | 138 |
| 75 | ++++ | 132 |
| 37,5 | ++++ | 129 |
| 18 | ++++ | 106 |
| 9 | ++ | 86 |
| 4,5 | + | 50 |
| 2,25 | - | 22 |

Tabelle 2

| Anti-Streptolysin-O (IU/ml) | Reflektrometrie bei 660 nm (Bits) |
|---|---|
| 150 | 94 |
| 37,5 | 78 |
| 18 | 56 |
| 9 | 38 |
| 4,5 | 31 |
| 2,25 | 23 |

0196731

1. Verfahren zum Nachweis und zur Bestimmung der Konzentration eines in wässriger Lösung vorliegenden Analyten mit bioaffinen Bindungseigenschaften durch Agglutination oder durch Agglutinationshemmung von Partikeln, welche mit bioaffinen Bindungseigenschaften und zugleich mit einem signalproduzierenden System (SPS) oder mit Teilen eines SPS ausgestattet sind, dadurch gekennzeichnet, daß

   a) die Lösung des Analyten auf einen Bereich einer Vorrichtung aufgebracht wird, der als Auftragszone ausgestattet ist, wobei die Lösung anschließend Bereiche durchwandert, die in genannter Reihenfolge als Reaktions, Trenn-und Nachweiszone ausgestattet sind,

   b) eine Trennung von agglutinierten und nicht agglutinierten Partikeln vorgenommen wird mit Hilfe dieser Vorrichtung bestehend aus Materialien, die wässrige Lösungen und darin dispergierte Partikel aufsaugen und transportieren und

   c) der abgetrennte Anteil von nicht agglutinierten Partikeln über sein SPS bestimmt wird.

2. Verfahren nach Anspruch 1, dadurch gekennzeichnet, daß

    a) der Analyt in der Reaktionszone eine Agglutination von Partikeln, welche mit bioaffinen Bindungseigenschaften und zugleich mit einem SPS oder Teilen eines SPS ausgestattet sind, hervorruft oder verhindert

    b) die nicht agglutinierten Partikel die Trennzone durchwandern und in die Nachweiszone gelangen und

    c) die nicht agglutinierten Partikel in der Nachweiszone mittels des SPS nachgewiesen und bestimmt werden.

3. Verfahren nach Anspruch 1, dadurch gekennzeichnet, daß in wässriger Lösung dispergierte Partikel mit bioaffinen Bindungseigenschaften auf die Reaktionszone aufgetragen werden zu einem Zeitpunkt, der vor dem Auftragen der die nachzuweisende Komponente enthaltende Lösung auf die Auftragszone liegt oder mit diesem zusammenfällt.

4. Verfahren nach Anspruch 1, dadurch gekennzeichnet, daß eine Vorrichtung verwendet wird, die in der Auftrags-oder in der Reaktionszone, oder auf beide Zonen verteilt, Partikel mit bioaffinen Bindungseigenschaften in trockener Form enthalten.

5. Verfahren nach Anspruch 1, dadurch gekennzeichnet,
daß eine Vorrichtung verwendet wird, welche in der
Nachweiszone Bestandteile eines SPS enthalten, die
zusammen mit den in die Nachweiszone gewanderten
Partikeln ein meßbares Signal erzeugen.

6. Verfahren nach Anspruch 1, dadurch gekennzeichnet,
daß eine Vorrichtung verwandt wird, in der die Auftrags-und Reaktionszone zu einem einzigen Bereich
zusammengefaßt sind.

7. Vorrichtung zum Nachweis und zur Bestimmung der Konzentration eines in wässriger Lösung vorliegenden
Analyten mit bioaffinen Bindungseigenschaften mittels
Partikeln in einer Agglutinationsreaktion, wobei agglutinierte und nicht agglutinierte Anteile voneinander getrennt werden, dadurch gekennzeichnet, daß

a) die Vorrichtung eine Auftrags-, eine Reaktions-,
eine Trenn- und eine Nachweiszone enthält,

b) alle Zonen aus Materialien bestehen, die wässrige
Flüssigkeiten aufnehmen und transportieren,

c) alle genannten Zonen für die nicht agglutinierten
Partikel durchlässig sind,

d) zumindest die Trennzone für agglutinierte Partikel
undurchlässig ist,

0196731

e) die Vorrichtung ein signalproduzierendes System
   enthält, welches in der Nachweiszone ein auswertbares Signal erzeugt.

8. Vorrichtung nach Anspruch 7, dadurch gekennzeichnet,
   daß sie agglutinationsfähige Partikel in trockener
   Form enthält.

9. Vorrichtung nach Anspruch 7, dadurch gekennzeichnet,
   daß entweder

   a) an die Partikel ein bioaffiner Bindungspartner
      des Analyten und mindestens ein Bestandteil des
      SPS gebunden sind, oder

   b) an die Partikel eine vorgegebene Menge des Analyten oder eines Derivats des Analyten und mindestens
      ein Bestandteil des SPS gebunden sind, wobei ein
      mindestens bifunktioneller Bindungspartner des
      Analyten in der Auftragszone oder der Reaktionszone vorhanden ist, oder

   c) an die Partikel ein bioaffiner Bindungspartner
      des Analyten und mindestens ein Bestandteil des
      SPS gebunden sind, wobei in der Auftrags- oder Reaktionszone ein zumindest bifunktionelles Derivat des
      Analyten vorhanden ist.

FIG.1

FIG.2

FIG.3

FIG.4

0196731
Nummer der Anmeldung

**Europäisches Patentamt**

## EUROPÄISCHER RECHERCHENBERICHT

| EINSCHLÄGIGE DOKUMENTE | | | EP 86200566.7 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| Y | EP - A2 - 0 119 623 (FUJI PHOTO FILM CO., LTD.)<br>* Ansprüche 1,24 *<br>-- | 1,7 | G 01 N 33/549<br>G 01 N 33/52 |
| Y | DE - A1 - 3 210 080 (BEHRINGWERKE AG)<br>* Ansprüche 1-7 *<br>---- | 1,7 | |

| | RECHERCHIERTE SACHGEBIETE (Int. Cl.4) |
|---|---|
| | G 01 N 33/00 |

Der vorliegende Recherchenbericht wurde für alle Patentansprüche erstellt.

| Recherchenort | Abschlußdatum der Recherche | Prüfer |
|---|---|---|
| WIEN | 13-06-1986 | SCHNASS |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82